# EUROPEAN PATENT SPECIFICATION

(11) **EP 0 348 981 B1**
(45) Date of publication and mention of the grant of the patent: **05.01.1994**
(21) Application number: 89111878.8
(22) Date of filing: 29.06.1989
(51) Int. Cl.: C07C 43/17, C07C 21/19, C07C 21/215, C07C 41/24, C07C 17/24

(54) **Process for preparing fluorinated conjugated olefinic products**
Verfahren zur Herstellung von fluorierten konjugierten olefinischen Produkten
Procédé pour la préparation de produits fluorés conjugués oléfiniques

(30) Priority: 30.06.1988 IT 2116288
(43) Date of publication of application: 03.01.1990
(73) Proprietor: AUSIMONT S.p.A., I-20121 Milano (IT)
(72) Inventor: Maraccini, Antonio, I-28040 Dormelletto Novara (IT); Pasquale, Antonio, I-28100 Novara (IT); Fiorani, Tiziana, I-28100 Novara (IT)
(74) Representative: Barz, Peter, Dr.

(56) References cited:
- US-A- 2 705 247
- US-A- 2 745 885
- US-A- 2 777 004
- US-A- 2 894 042
- US-A- 3 046 304
- CHEMICAL ABSTRACTS, vol. 98, no. 16, 18th April 1983, pages 11-12, abstract no. 126788e, Columbus, Ohio, US; & CS-A-200 953 (V. DEDEK et al.) 01-02-1983

## Description

The present invention relates to a process for preparing conjugated fluorinated olefinic products and to the new products obtainable thereby.

US-A-3,046,304 is directed to the coupling of halogenated compounds and the use thereof for the preparation of, e.g., halogenated alkenes (such as fluorinated and chlorinated olefins). It also describes the dehalogenation of corresponding halogenated alkanes by means of zinc powder.

US-A-2,777,004 describes the preparation of hexafluorobutadiene from symmetrical dichlorodifluoroethylene by dechlorination with zinc powder.

US-A-2,745,885 discloses the dechlorination of 1,1,2,3,4-pentachloro-1,2,3,4,4-pentafluorobutane with zinc to yield 1-chloro-1,2,3,4,4-pentafluoro-1,3-butadiene.

US-A-2,894,042 is concerned with the preparation of hexafluorobutadiene through the reaction of 1,2,3,4-tetrachloroperfluorobutane with zinc.

According to CS-A-200,953 (Chem. Abstr. vol. 98, no. 16, 18 April 1983, abstract no. 126788e) polyenes are produced by dehalogenation of α,ω-dibromochlorofluoroalkanes with zinc powder.

It is one object of the present invention to provide an economically advantageous process for the preparation of certain perfluorinated conjugated olefins.

Another object of the present invention is the provision of fluorinated conjugated olefins which show perhaloalkoxy groups and/or perhaloalkyl groups and/or chlorine atoms at one or both ends of the molecule.

These and other objects are achieved by means of the process according to the present invention, according to which fluorinated conjugated olefinic products are obtained which have the formulae:

RₓO-(CF=CF)ₘ-F (A)

RₓO-(CF=CF)ₘ-ORₓ (B)

RₓO-(CF=CF)ₘ-Cl (E)

R_{y}-(CF=CF)ₘ-ORₓ (I)

wherein:
- Rₓ: is a linear or branched perfluoroalkyl radical having from 1 to 10 carbon atoms;
- R_{y}: is a linear or branched perhalogenated alkyl radical, a perhaloalkyl-monoether radical or a perhaloalkyl-polyether radical having from 1 to 9 carbon atoms and containing fluorine atoms or fluorine and chlorine atoms; and
- m: ranges from 2 to 10.

The present process is characterized in that telomeric products having the formulae

RₓO-(CFCl-CFCl)ₘ-F (A')

RₓO-(CFCl-CFCl)ₘ-ORₓ (B')

RₓO-(CFCl-CFCl)ₘ-Cl (E')

R_{y}-(CFCl-CFCl)ₘ-ORₓ (I')

wherein Rₓ, R_{y} and m have the meanings specified above, are reacted with zinc powder at temperatures of from about 40°C to about 160°C, preferably from about 40°C to about 100°C, and in a solvent selected from (preferably C₁₋₆) alcohols, dimethylformamide, dimethylsulfoxide.

When an alcohol is used as solvent, it may be selected from, for example, methanol, ethanol, propanol, isopropanol, isobutanol and mixtures thereof.

The reaction is usually carried out in the presence of a dechlorination activator known from the prior art, for example, KI or NaI.

A known agent, which acts as neutralizer of the possibly developed acidity such as, for example, K₂CO₃ or Na₂CO₃ is also commonly employed.

The reaction is suitably carried out at the reflux temperature of the solvent.

The telomeric products to be used as the starting materials can be prepared by means of a process disclosed in EP-A-348980.

The disclosure of said application is incorporated herein by reference.

According to said process, 1,2-dichloro-1,2-difluoroethylene is reacted, at a temperature of from -100°C to +50°C, with a perhalofluorooxy compound of formula

Rₓ-OF

wherein Rₓ has the meanings given above. Thereby, a mixture of telomers having different end groups and different degrees of telomerization is obtained.

In practising the above process, a gaseous stream of perhalofluorooxy compound, diluted with an inert gas, is usually fed to a reactor charged with 1,2-dichloro-1,2-difluoroethylene (in the liquid state or dissolved in an inert solvent). By using particular working conditions (such as, for example, the addition of F₂ to the reaction medium), the prevailing production of telomers with certain end groups and with an either comparatively low or a relatively high degree of telomerization can be favoured.

When the telomerization reaction is carried out in the absence of F₂ and Rₓ contains one carbon atom, the telomeric products of formulae (A') and (B') are prevailingly formed among others whereas, when Rₓ contains two or more carbon atoms, products of formulae (A'), (B') and (I') are among the predominant ones.

When the telomerization reaction is carried out in the presence of F₂, under particular conditions and at temperatures of from -100°C to about -60°C, the same telomeric products are obtained, with products (A') being among the predominant ones.

When the telomerization reaction is carried out in the presence of F₂, under different conditions and at temperatures of from about -60°C up to about -20°C, species of formula (E') are obtained, among others, together with those of formulae (A'), (B') and (I').

In the process according to the present invention the mixture of telomeric products is, preferably, used in the same state as it is obtained in, so that a mixture of fluorinated conjugated olefinic products which have a varying degree of telomerization, coincident with the telomerization degree of the starting products, is obtained at the end of the reaction.

As long as the degree of telomerization does not exceed 5, the individual olefinic products can be separated from one another by distillation.

In the telomeric products used as starting materials as well as in the olefinic products obtained, R_{y} preferably contains two oxygen atoms in case it represents perhaloalkyl-polyether radicals.

R_{y} preferably represents perfluorinated radicals.

Preferably, Rₓ contains from 1 to 6, particularly from 1 to 3, and R_{y} contains from 1 to 5, particularly from 1 to 3, carbon atoms.

When R_{y} contains chlorine atoms, it preferably does not comprise any -CCl₃ or -CFCl-CFCl- groups.

In both the starting products and the end products, m preferably ranges from 2 to 5.

The olefinic products obtainable by means of the process according to the present invention may be used as comonomers for the preparation of polymers. These polymers, by having been obtained from conjugated olefins, may contain double bonds in the branches of the main chain (backbone) of the polymer. Such double bonds can form sites for crosslinking or other kinds of reactions. The presence of -ORₓ or R_{y} groups results in polymeric products with properties different from those of polymers obtained from perfluorinated conjugated olefins not carrying such groups. Furthermore, the present olefinic products may be used for the preparation of dielectric fluids and heat exchange fluids.

In the process according to the present invention the ratio of gram-atoms Zn to the gram-atoms of units of the formula

-CFCl-CFCl-

is usually within the range of from 1.00 to 1.5, and preferably of from 1.00 to 1.05. The molar ratio of Zn to activator compound, calculated on the basis of KI, generally ranges from 10 to 37. The molar ratio of Zn to agent for the neutralization of acidity, calculated on the basis of K₂CO₃, is generally within the range of from 4 to 13.

In the process according to the present invention, in addition to the fluorinated conjugated olefinic products small amounts of non-conjugated, chloro-fluorinated olefinic products may be formed, which products contain, for example, the sequence

-CFCl-CF=CF-CFCl-

The desired fluorinated conjugated olefinic products can easily be separated from the above chloro-fluorinated products by distillation.

When the process according to the invention is carried out with a mixture of telomeric products obtained by means of the use of CF₃OF (as telogen) in the reaction described in EP-A-348980 mentioned above, which mixture is predominantly composed of the following telomeric species:

F-(CFCl-CFCl)ₘ-F (IIIa)

CF₃O-(CFCl-CFCl)ₘ-F (Ia)

and

CF₃O-(CFCl-CFCl)ₘ-OCF₃ (IIa)

the following olefinic products will prevailingly be formed:

F-(CF=CF)ₘ-F (IIIf)

CF₃O-(CF=CF)ₘ-F (If)

and

CF₃O-(CF=CF)ₘ-OCF₃ (IIf)

The species (If) and (IIf) are new products. The product (IIIf), with m equal to 2, is perfluorobutadiene.

When a mixture of telomeric products, obtained by means of the use of CF₃CF₂OF as telogen in the telomerization reaction and primarily composed of the following telomeric species:

CF₃CF₂O-(CFCl-CFCl)ₘ-F (Ic)

CF₃CF₂O-(CFCl-CFCl)ₘ-OCF₂CF₃ (IIc)

CF₃-(CFCl-CFCl)ₘ-F (IIIc)

CF₃-(CFCl-CFCl)ₘ-OCF₂CF₃ (IVc)

and

F-(CFCl-CFCl)ₘ-F (IIIa)

is used as starting material, the following olefinic products will prevailingly be formed:

CF₃CF₂O-(CF=CF)ₘ-F (I′c)

CF₃CF₂O-(CF=CF)ₘ-OCF₂CF₃ (II′c)

CF₃-(CF=CF)ₘ-F (III′c)

CF₃-(CF=CF)ₘ-OCF₂CF₃ (IV′c)

The species (I′c), (II′c), (III′c) and (IV′c) are new products.

When a mixture of telomeric products obtained by means of the use of CF₃-CF₂-CF₂-OF as telogen in the telomerization reaction and predominantly composed of the following telomeric species:

CF₃CF₂CF₂O-(CFCl-CFCl)ₘ-F (Id)

CF₃CF₂CF₂O-(CFCl-CFCl)ₘ-OCF₂CF₂CF₃ (IId)

CF₃CF₂CF₂O-(CFCl-CFCl)ₘ-OCF₂CF₃ (IIId)

and

F-(CFCl-CFCl)ₘ-F (IIIa)

is used as starting material, the following olefinic products will prevailingly be formed:

CF₃CF₂CF₂O-(CF=CF)ₘ-F (I'd)

CF₃CF₂CF₂O-(CF=CF)ₘ-OCF₂CF₂CF₃ (II'd)

CF₃CF₂CF₂O-(CF=CF)ₘ-OCF₂CF₃ (III'd)

and

F-(CF=CF)ₘ-F (III'f)

The species (I'd), (II'd) and (III'd) are new products.

The main advantages of the present invention can be summarized as follows:
- New fluorinated conjugated olefins, which show perhaloalkoxy groups and/or perhaloalkyl groups at one or both ends of the molecular chain, may be obtained.

The following examples are given in order to illustrate the present invention without limiting it.

### EXAMPLE 1

The mixture of telomers used as starting material was prepared as follows:
A gaseous stream of 0.6 Nl/hour of fluorooxy-trifluoromethane, 0.6 Nl/hour of F₂ and 103 Nl/hour of N₂ was bubbled through 450 g of liquid 1,2-dichloro-1,2-difluoro-ethylene (DCDFE), cooled to -72°C in a reactor equipped with condenser, thermometer and stirrer.

After 18 hours the reaction was discontinued and unreacted DCDFE was distilled off.

105 g of product were recovered, which product was analysed by gas-chromatography on a 3% SP 2100 column, gas mass and ¹⁹F-N.M.R. spectroscopy.

The product was composed prevailingly of the following species:

CF₃O-(CFCl-CFCl)ₘ-F (Ia)

CF₃O-(CFCl-CFCl)ₘ-OCF₃ (IIa)

and

F-(CFCl-CFCl)ₘ-F (IIIa)

The species in which m is 2 constituted 98% by weight of the mixture. Those in which m is 3 accounted for about 2% of the mixture.

Based on 100 g of mixture, the species were distributed as follows:
- (IIIa) = 87 g
- (Ia) = 4.8 g
- (IIa) = 3.6 g
A reactor equipped with thermometer, stirrer, dropping funnel and collection and rectification system was charged with 210 ml of dimethylformamide and, subsequently, 50 g of Zn powder, 10 g of K₂CO₃ and 7 g of KI were added thereto.

Thereupon, 100 g of the above telomeric mixture were successively introduced within 4 hours.

The dechlorination reaction started at a temperature of 40°C. During the dropwise addition the temperature was kept from exceeding 60°C. Upon completion of the addition the reaction was completed by maintaining the reaction mixture at 55 to 60°C for 1 hour and then at about 68°C for a further hour.

During the feeding of the telomers and the completion of the reaction, 31 g of the component of formula

F-(CF=CF)₂-F

i.e., perfluorobutadiene, were collected in the reactor rectification system.

At the end of the perfluorobutadiene collection the residual reaction mixture was subjected to fractional distillation.

Collected were:
- 0.5 g of CF₃O-(CF=CF)₂-OCF₃ in the form of three positional isomers;
- Traces of CF₃O-(CF=CF)₂-F in the form of 2 positional isomers;
- 0.7 g of CF₃O-CF=CF-CCl=CF₂ in the form of 3 positional isomers.

The reaction products were analysed by gas-chromatography on a 1% SP 1000 column, gas mass and ¹⁹F-N.M.R. spectroscopy.

The isomers of formula

CF₃O-CF=CF-CCl=CF₂

result from the presence, in the telomers used as the starting products, of isomers of formula

CF₃O-CFCl-CFCl-CFCl=CF₂.

### EXAMPLE 2

The mixture of telomers used as the starting product (100 g) was prevailingly composed of the following species:

| | |
|---|---|
| a) F-(CFCl-CFCl)ₘ-F | 30.4 g |
| b) CF₃O-(CFCl-CFCl)ₘ-F and CF₃O-[(CFCl-CFCl)_{m-½}-CHF]-F, totalling | 26.5 g |
| c) CF₃O-(CFCl-CFCl)ₘ-OCF₃ | 31.5 g and |
| d) CF₃O-(CFCl-CFCl)ₘ₋₁-CCl=CF₂ | 9.4 g. |

The species in which m is 2 constituted 95% by weight of the mixture. Those in which m is 3 accounted for 5% of said mixture.

A reactor equipped with thermometer, stirrer, dropping funnel and collection and rectification system was charged with 210 ml of dimethylformamide and, subsequently, 50 g of Zn powder, 10 g of K₂CO₃ and 7 g of KI were added thereto.

Thereupon, 100 g of the above telomeric products were successively introduced within 4 hours.

The dechlorination reaction started at 40°C and the temperature was kept from exceeding 60°C. Upon completion of the feeding step the reaction was completed by maintaining the reaction mixture at 55 to 60°C for 1 hour, and then at 68°C for a further 1.5 hours.

Already during the introduction of the telomers and also during the completion of the reaction 11.1 g of perfluorobutadiene were collected in the reactor rectification system.

After the collection of the perfluorobutadiene the residual reaction mixture was subjected to fractional distillation.

Collected were:
- 6.1 g of CF₃O-(CF=CF)₂-F in the form of 2 positional isomers;
- 12.1 g of CF₃O-(CF=CF)₂-OCF₃ in the form of 3 positional isomers;
- 9.2 g of CF₃O-CF=CF-CCl=CF₂ in the form of 3 positional isomers.

The products obtained were analysed by the methods described in example 1.

### EXAMPLE 3

The mixture of telomers used as starting material (60 g) was prevailingly composed of the following species:

| | |
|---|---|
| a) F-(CFCl-CFCl)ₘ-F | 25.4 g; |
| b) CF₃-(CFCl-CFCl)ₘ-F CF₃O-[(CFCl-CFCl)_{m-½}-CHF]-F and CF₃O-(CFCl-CFCl)ₘ₋₁-CCl=CF₂, totalling | 19.3 g; |
| c) CF₃O-(CFCl-CFCl)ₘ-OCF₃ | 12.1 g. |

The species in which m is 2 constituted about 99% by weight of the mixture.

A reactor equipped with thermometer, stirrer, dropping funnel and collection and rectification system was charged with 150 ml of isobutanol and, subsequently, 31.5 g of Zn powder, 6.5 g of K₂CO₃ and 3.5 g of KI were added thereto.

Thereupon, 60 g of the above telomeric mixture were sucessively introduced within 3 hours.

The dechlorination reaction started at a temperature of 85°C. After the reaction the temperature was caused to decrease to about 60°C. After the feeding of the telomers, the reaction was completed by maintaining the reaction mixture at 65 to 70°C for 1 hour, and then at a maximum temperature of 80°C for a further 1.5 hours.

During the introduction of the telomers and during the completion of the reaction, 8.5 g of perfluorobutadiene were collected.

When the collection of perfluorobutadiene was completed, the residual reaction mixture was subjected to fractional distillation.

Collected were:
- 6.1 g of CF₃O-(CF=CF)₂-F in the form of 2 positional isomers;
- 2.1 g of CF₃O-CF=CF-CCl=CF₂ in the form of 3 positional isomers;
- 2.85 g of CF₃O-(CF=CF)₂-OCF₃ in the form of 3 positional isomers.

The products obtained were analysed by the methods described in example 1.

## Claims

1. Fluorinated conjugated olefinic products having one or more of the formulae:
RₓO-(CF=CF)ₘ-F (A)
RₓO-(CF=CF)ₘ-ORₓ (B)
RₓO-(CF=CF)ₘ-Cl (E)
R_{y}-(CF=CF)ₘ-ORₓ (I)
wherein:
Rₓ is a linear or branched perfluoroalkyl radical having from 1 to 10 carbon atoms;
R_{y} is a linear or branched perhaloalkyl, perhaloalkyl-monoether or perhaloalkylpolyether radical having from 1 to 9 carbon atoms and containing fluorine atoms or fluorine and chlorine atoms and
m ranges from 2 to 10.

2. Products according to claim 1, wherein Rₓ contains from 1 to 6 carbon atoms and R_{y} contains from 1 to 5 carbon atoms.

3. Products according to one or more of the preceding claims wherein m ranges from 2 to 5.

4. Process for preparing the products according to claim 1, wherein products having the formulae
RₓO-(CFCl-CFCl)ₘ-F (A')
RₓO-(CFCl-CFCl)ₘ-ORₓ (B')
RₓO-(CFCl-CFCl)ₘ-Cl (E')
R_{y}-(CFCl-CFCl)ₘ-ORₓ (I')
wherein Rₓ, R_{y} and m are defined as in claim 1, are reacted with zinc powder at temperatures of from about 40°C to about 160°C and in a solvent selected from alcohols, dimethylformamide and dimethylsulfoxide.

5. Process according to claim 4, characterized in that the alcoholic solvent is selected from methanol, ethanol, propanol, isopropanol and isobutanol.

6. Process according to claim 4 or 5, characterized in that the reaction is carried out in the presence of a dechlorination activator.

7. Process according to one or more of claims 4 to 6, characterized in that the reaction is carried out in the presence of an agent which acts as neutralizer of acidity.

## Patentansprüche

1. Fluorierte konjugierte Olefin-Produkte mit einer oder mehreren der Formeln:
RₓO-(CF=CF)ₘ-F (A)
RₓO-(CF=CF)ₘ-ORₓ (B)
RₓO-(CF=CF)ₘ-Cl (E)
R_{y}-(CF=CF)ₘ-ORₓ (I)
worin:
Rₓ ein linearer oder verzweigter Perfluoralkylrest mit 1 bis 10 Kohlenstoffatomen ist;
R_{y} ein linearer oder verzweigter Perhalogenalkyl-, Perhalogenalkylmonoether- oder Perhalogenalkylpolyether-Rest mit 1 bis 9 Kohlenstoffatomen, der Fluoratome oder Fluor- und Chloratome enthält, ist und
m im Bereich von 2 bis 10 liegt.

2. Produkte nach Anspruch 1, in welchen Rₓ 1 bis 6 Kohlenstoffatome enthält und R_{y} 1 bis 5 Kohlenstoffatome enthält.

3. Produkte nach einem oder mehreren der vorangehenden Ansprüche, in welchen m im Bereich von 2 bis 5 liegt.

4. Verfahren zur Herstellung der Produkte nach Anspruch 1, in welchem Produkte mit den Formeln
RₓO-(CFCl-CFCl)ₘ-F (A')
RₓO-(CFCl-CFCl)ₘ-ORₓ (B')
RₓO-(CFCl-CFCl)ₘ-Cl (E')
R_{y}-(CFCl-CFCl)ₘ-ORₓ (I')
worin Rₓ, R_{y} und m wie in Anspruch 1 definiert sind, bei Temperaturen von etwa 40°C bis etwa 160°C und in einem aus Alkoholen, Dimethylformamid und Dimethylsulfoxid ausgewählten Lösungsmittel mit Zinkpulver umgesetzt werden.

5. Verfahren nach Anspruch 4, dadurch gekennzeichnet, daß das Alkohol-Lösungsmittel aus Methanol, Ethanol, Propanol, Isopropanol und Isobutanol ausgewählt wird.

6. Verfahren nach Anspruch 4 oder 5, dadurch gekennzeichnet, daß die Umsetzung in Anwesenheit eines Dechlorierungsaktivators durchgeführt wird.

7. Verfahren nach einem oder mehreren der Ansprüche 4 bis 6, dadurch gekennzeichnet, daß die Umsetzung in Anwesenheit eines Mittels, das als Neutralisationsmittel für Acidität wirkt, durchgeführt wird.

## Revendications

1. Produits oléfiniques conjugués et fluorés représentés par l'une ou plusieurs des formules suivantes:
RₓO-(CF=CF)ₘ-F (A)
RₓO-(CF=CF)ₘ-ORₓ (B)
RₓO-(CF=CF)ₘ-Cl (E)
R_{y}-(CF=CF)ₘ-ORₓ (I)
dans lesquelles:
Rₓ représente un radical perfluoroalkyle linéaire ou ramifié comportant de 1 à 10 atomes de carbone;
R_{y} représente à un radical alkyle perhalogéné, un radical perhaloalkyl-monoéther ou un radical perhaloalkylpolyéther linéaire ou ramifié, comportant de 1 à 9 atomes de carbone et contenant des atomes de chlore ou des atomes de fluor et de chlore; et
m varie de 2 à 10.

2. Produits selon la revendication 1, caractérisés en ce que Rₓ contient de 1 à 6 atomes de carbone et en ce que R_{y} contient de 1 à 5 atomes de carbone.

3. Produits selon une ou plusieurs des revendications précédentes, caractérisés en ce que m varie de 2 à 5.

4. Procédé de préparation des produits selon la revendication 1, caractérisé en ce que les produits représentés par les formules:
RₓO-(CFCl-CFCl)ₘ-F (A')
RₓO-(CFCl-CFCl)ₘ-ORₓ (B')
RₓO-(CFCl-CFCl)ₘ-Cl (E')
R_{y}-(CFCl-CFCl)ₘ-ORₓ (I')
dans lesquelles Rₓ, R_{y} et m sont tels que définis dans la revendication 1, réagissent avec une poudre de zinc à des températures comprises entre environ 40°C et environ 160°C, dans un solvant sélectionné parmi les alcools, le diméthylformamide et le diméthylsulfoxyde.

5. Le procédé selon la revendication 4, caractérisé en ce que le solvant alcoolique est sélectionné parmi méthanol, éthanol, propanol, isopropanol et isobutanol.

6. Le procédé selon la revendication 4 ou 5, caractérisé en ce que la réaction est mise en oeuvre en présence d'un activateur de déchloration.

7. Le procédé selon l'une ou plusieurs des revendications 4 à 6, caractérisé en ce que la réaction est mise en oeuvre en présence d'un agent neutralisant l'acidité.
